# FASCICULE DE BREVET EUROPEEN

(11) **EP 1 626 623 B1**
(45) Date de publication et mention de la délivrance du brevet: **21.07.2010**
(21) Numéro de dépôt: 04742815.6
(22) Date de dépôt: 24.05.2004
(51) Int. Cl.: A01K 67/027, C12N 5/07

(54) **PROCEDE DE CLONAGE DU RAT PAR TRANSFERT NUCLEAIRE**
KLONIERVERFAHREN BEI DER RATTE MITTELS ZELLKERNTRANSFER
METHOD FOR CLONING IN THE RAT BY NUCLEAR TRANSFER

(30) Priorité: 23.05.2003 FR 0306223
(43) Date de publication de la demande: 22.02.2006
(73) Titulaire: INSTITUT NATIONAL DE LA RECHERCHE AGRONOMIQUE, 75007 Paris (FR)
(72) Inventeur: COZZI, Jean, F-69007 Lyon (FR); ZHOU, Qi, HaiDian District, Beijing 100080 PR (CN); RENARD, Jean-Paul, F-92170 Vanves (FR)
(74) Mandataire: Tetaz, Franck Claude Edouard
(86) Numéro de dépôt international: PCT/FR2004/001275
(87) Numéro de publication internationale: WO 2004/105477

(56) Documents cités:
- WO-A-02/051997
- HIRABAYASHI M, KATO M, TAKEUCHI A, ISHIKAWA A, HOCHI S.: "Factors affecting premature chromosome condensation of cumulus cell nuclei injected into rat oocytes." J REPROD DEV. 2003 APR;49(2):121-6., vol. 49, no. 2, avril 2003 (2003-04), pages 121-126, XP0001190855 cité dans la demande
- HAYES E ET AL: "NUCLEAR TRANSFER OF ADULT AND GENETICALLY MODIFIED FETAL CELLS OF THE RAT" PHYSIOLOGICAL GENOMICS, AMERICAN PHYSIOLOGICAL SOCIETY, BETHESDA, MD, US, vol. 5, no. 4, 2001, pages 193-204, XP008004084 ISSN: 1094-8341 cité dans la demande
- IANNACCONE P ET AL: "PREIMPLANTATION AND POSTIMPLANTATION DEVELOPMENT OF RAT EMBRYOS CLONED WITH CUMULS CELLS AND FIBROBLASTS" ZYGOTE, CAMBRIDGE UNIVERSITY PRESS, CAMBRIDGE, GB, vol. 9, no. 2, mai 2001 (2001-05), pages 135-143, XP008009807 ISSN: 0967-1994 cité dans la demande
- JOSEFSBERG LB, GALIANI D, DANTES A, AMSTERDAM A, DEKEL N.: "The proteasome is involved in the first metaphase-to-anaphase transition of meiosis in rat oocytes." BIOL REPROD., vol. 62, no. 5, mai 2000 (2000-05), pages 1270-1277, XP002277913 cité dans la demande
- ZERNICKA-GOETZ, M.: "Spontaneous and induced activation of rat oocytes" MOLECULAR REPRODUCTION AND DEVELOPMENT, vol. 28, 1991, pages 169-176, XP0008030097 cité dans la demande
- LEE DO HEE ET AL: "Proteasome inhibitors: Valuable new tools for cell biologists" TRENDS IN CELL BIOLOGY, vol. 8, no. 10, octobre 1998 (1998-10), pages 397-403, XP002305191 ISSN: 0962-8924
- QI ZHOU, ET AL.: "Generation of Fertile Cloned Rats by Regulating Oocyte Activation" SCIENCE, VOL 302, ISSUE 5648, 1179, 14 NOVEMBER 2003, ORIGINALLY PUBLISHED IN SCIENCE EXPRESS ON 25 SEPTEMBER 2003, 14 novembre 2003 (2003-11-14), XP002277914

## Description

La présente invention concerne un procédé de clonage du rat par transfert nucléaire. La présente description concerne également les rats ainsi obtenus, ainsi que leur utilisation pour la production de molécules d'intérêt ou comme modèles d'étude.

Associée à une modification génétique des cellules avant leur utilisation comme source de noyaux, la technologie de transfert nucléaire permet l'établissement de lignées d'animaux génétiquement modifiés pour des caractéristiques particulières. Les techniques de transfert nucléaire sont bien connues et ont été développées pour le clonage de plusieurs espèces animales, en particulier de mammifères, comme le mouton (Wilmut *et al.,* 1997 ; WO 97 07669), la souris (Wakayama *et al.,* 1998 ; WO 99 37143), les bovins (Wells *et al.,* 1999), la chèvre (Baguisi *et al.,* 1999 ; WO 00 25578),le porc (Polejaeva *et al.,* 2000) et le lapin (Chesne et al., 2002). Les méthodes de transfert nucléaire sont notamment décrites par Campbell & al. (Nuclear transfer in practice, School of Biosciences, University of Nottingham, Leicestershire, United Kingdom).

Ces méthodes de transfert nucléaire comprennent généralement les étapes suivantes de :
a) prélèvement d'un ovocyte et son maintien dans un milieu de culture approprié ;
b) l'énucléation de cet ovocyte, c'est à dire l'enlèvement par micromanipulation de son noyau alors sous forme de métaphase
c) insertion d'un noyau de cellule somatique foetale ou adulte ou de cellule embryonnaire ou de cellule de la lignée germinale dans l'ovocyte énuclée reconstitué (transfert nucléaire)
d) activation de l'ovocyte reconstitué obtenu à l'étape c) pour obtenir un embryon reconstitué.

L'embryon reconstitué est ensuite soit implanté dans une femelle receveuse pour qu'il se développe, éventuellement en foetus puis en nouveau né, soit cultivé in vitro pendant les premiers stades de son développement préimplantatoire avant d'être placé dans l'oviducte ou l'utérus d'une femelle receveuse.

Le stade du cycle cellulaire atteint par l'ovocyte au moment de sa récolte et sa capacité à être maintenu à ce stade jusqu'au moment du transfert de noyau est un facteur limitant pour permettre non seulement l'énucléation efficace de l'ovocyte mais aussi la synchronisation entre les cycles cellulaires de l'ovocyte receveur et du noyau exogène et par conséquence l'obtention d'un embryon et de son développement à terme.

Le transfert nucléaire dans les ovocytes de rats a déjà été décrit dans la littérature scientifique (lannacone & al., 2001 ; Hayes & al., 2001 ; Hirabayashi & al., 2003 ; Hirabayashi & al., 2003). Toutefois, aucun transfert nucléaire permettant le développement d'un embryon implanté n'a été rapporté, ni évidemment le développement d'un foetus de rat puis d'un nouveau né à partir de ces ovocytes reconstitués.

Le procédé de clonage selon l'invention est le premier rapporté qui aura permis la production d'ovocytes de rat reconstitués capables de se développer à en foetus viables, et aussi en rats vivants dont la fertilité a pu être contrôlée.

Jusqu'à présent le maintien *in vitro* des ovocytes à l'état "non activé" c'est à dire au stade de seconde division de méiose était assuré chez les mammifères et particulièrement chez le rat par le prélèvement rapide des oviductes hors de l'animal sacrifié puis la préparation et le maintien in vitro des ovocytes dans un environnement approprié (température, pH, calcium) (exposition à un milieu de culture dépourvu de calcium par exemple) qui ne fait que retarder l'évolution du processus d'activation ovocytaire. Ce processus qui est déclenché dans les conditions naturelles par le spermatozoïde est caractérisé par une libération pulsatile d'ions calcium stockés dans des vésicules de l'endoplasme conduisant à une diminution brutale de l'activité du facteur MPF (Maturation Promoting Factor), un complexe protéique caractérisé par l'association de la cycline B et d'une enzyme kinase (cdc2), par dégradation protéolytique des cyclines impliquées. Les inconvénients majeurs des approches utilisées jusqu'à ce jour et citées précédemment pour maintenir l'ovocyte au stade de métaphase de seconde division méiotique sont d'une part l'impossibilité d'inhiber efficacement l'évolution biochimique *in vitro* de l'ovocyte et d'autre part de maintenir l'ovocyte dans un état physiologique lui permettant de supporter les contraintes mécaniques de la micromanipulation. En effet cette évolution biochimique devient rapidement défavorable dans le cas de transfert de noyaux après énucléation de l'ovocyte (clonage) car elle conduit rapidement à l'apparition d'une grande fragilité membranaire de l'ovocyte et donc à sa lyse plus fréquente après micromanipulation, et surtout à une diminution du potentiel de développement de l'embryon reconstitué car son noyau subit alors rapidement des remaniements inhérents à l'entrée en interphase qui accompagne le déclenchement du processus d'activation. Le processus d'activation lorsqu'il se produit de façon spontanée avant le transfert de noyau peut avoir 2 conséquences défavorables pour la reprogrammation du noyau exogène :
- d'abord, des déformations très rapides de la chromatine exposée dès son introduction dans l'ovocyte au processus dynamique de décondensation qui entraîne des altérations structurales nombreuses et la formation de plusieurs micro-noyaux ;
- ensuite l'exposition du noyau à un environnement cytoplasmique dans lequel des facteurs requis pour la reprogrammation sont dégradés empêchant la formation d'un noyau fonctionnel.

Bien que ces facteurs ne soient pas connus, leur importance est clairement démontrée par le fait qu'aucune reprogrammation ne peut être obtenue quand on introduit le noyau dans un oeuf au stade une cellule (donc déjà activé) et énucléé.

Le procédé selon l'invention permet le maintien in vitro de l'ovocyte dans un état physiologique stable pendant plusieurs heures après sa collecte de l'oviducte et ainsi prévenir l'activation spontanée in vitro de l'ovocyte observée chez beaucoup d'espèces mais plus particulièrement chez le rat (Zernicka-Goetz, 1990). Elle permet d'exposer le noyau exogène à un environnement cytoplasmique qui favorise l'organisation de sa chromatine en une structure de type métaphasique semblable à celle du noyau de l'ovocyte auquel il a été substitué. Le procédé selon l'invention peut également comprendre une technique rapide de reconstruction de l'embryon propice à sa réactivation en permettant (i) de réduire le temps d'exposition de l'ovocyte aux composés qui en préviennent l'activation et (ii) d'augmenter le temps d'exposition de la chromatine exogène aux facteurs ovocytaires impliqués dans la reprogrammation fonctionnelle d'un noyau encore présents et/ou fonctionnels dans le cytoplasme avant l'activation.

La présente invention concerne donc un procédé *in vitro* de production d'embryons de rats reconstitués par transfert nucléaire comprenant les étapes suivantes :
a) prélèvement d'un ovocyte de ratte et maintien dans un milieu approprié ;
b) insertion d'un noyau de cellule de rat dans l'ovocyte receveur de ratte précédemment maintenu in vitro pour obtenir un ovocyte reconstitué (transfert nucléaire) ;
c) activation de l'ovocyte reconstitué obtenu à l'étape b) pour obtenir un embryon reconstitué ;
   caractérisé en ce que
   - l'ovocyte receveur de ratte prélevé est maintenu à un stade métaphasique de la seconde division méiotique par maintien dans un milieu approprié comprenant un inhibiteur réversible du protéasome (milieu de maintien), et en ce que
   - l'ovocyte reconstitué est activé par exposition dans un milieu approprié contenant un inducteur de l'activation (milieu d'activation).

Les milieux appropriés pour le maintien in vitro et/ou pour l'activation des ovocytes, en particulier des ovocytes de rat, sont bien connus de l'homme du métier, notamment décrits dans les références citées précédemment ainsi que par Miyoshi et al. (1997, Stage dépendant development of rat 1-cell embryos in a chemically defined médium after fertilization in vivo and in vitro. Biol. Reprod. ; 56 : 180-185).

Les inhibiteurs réversibles du protéasome sont également connus de l'homme du métier, notamment décrits par Hee Lee & Goldberg (Profeasome inhibitors : valuable new tools for cell biologists, Trends in Cell Biology (Vol. 8) October 1998). Ils sont avantageusement choisis parmi le peptide aldéhyde Cbz-LLL-H (MG132), le peptide boronate Cbz-LLL-B(OH)2, le vinyl sulfone Cbz-LLL-Vs, la lactacystine et la bêta-lactone, la leupeptine, le MG101, le MG115, le MG262 et le PSI (Cbz-ile-glu), de préférence le MG132.

Sans préjuger de leur mode de fonctionnement, le caractère réversible de l'action de certains inhibiteurs du protéasome pourra être obtenu en limitant le temps d'exposition des ovocytes récepteurs récoltés et maintenus in vitro à ces inhibiteurs du protéasome.

Le temps d'exposition maximum pendant lequel les ovocytes récoltés pourront être maintenus in vitro à un stade métaphasique de la seconde division méiotique sans perdre leur capacité à être activés pour le développement d'embryons, puis de foetus et enfin de rats nouveaux nés viables, pourra être déterminé de manière expérimentale par l'homme du métier.

De manière préférentielle, ce temps d'exposition de l'ovocyte receveur à l'inhibiteur réversible du protéasome dans le milieu de maintien est inférieur à 8 heures, de préférence compris entre 1 et 6 heures.

La quantité d'inhibiteur réversible du protéasome dans le milieu de maintien de l'ovocyte récepteur sera déterminée par l'homme du métier en fonction des propriétés spécifiques de chaque inhibiteur, de manière qu'il permette le maintien réversible de l'ovocyte au stade métaphasique de la seconde méiose. La concentration de cet inhibiteur dans le milieu maintien sera de préférence comprise entre 1 et 10µM.

Pour l'étape b) de transfert nucléaire, le milieu de culture de l'ovocyte receveur de ratte est substantiellement exempt d'inhibiteur réversible du protéasome. Ceci permet d'éviter l'exposition des cellules donneuses de noyau à l'action desdits inhibiteurs et un potentiel effet négatif sur la capacité des ovocytes reconstitués à développer ensuite des embryons fonctionnels, puis des foetus fonctionnels et des nouveaux nés.

Le transfert nucléaire comprend généralement une étape d'enlèvement du noyau de l'ovocyte receveur, suivi d'une étape d'introduction du noyau de cellule donneuse dans l'ovocyte énucléé.

Selon un mode préférentiel de réalisation de l'invention, le transfert nucléaire comprend une étape d'introduction du noyau de cellule donneuse dans l'ovocyte receveur suivi d'une étape d'enlèvement du noyau de l'ovocyte receveur.

Le transfert du noyau de cellule donneuse dans le cytoplasme de l'ovocyte receveur est préférentiellement effectué par micro-injection. L'enlèvement du noyau de l'ovocyte receveur est préférentiellement effectué par micropipetage.

Selon un mode plus préférentiel de réalisation de l'invention, le transfert nucléaire est effectué par micro-injection avec reconstruction rapide de l'ovocyte en une seule étape.

Les techniques conventionnelles de reconstruction des embryons procèdent généralement en 2 étapes. Elles consistent dans un premier temps à énucléer l'ovocyte puis à incorporer dans le cytoplasme énucléé le noyau désiré par micro-injection ou électrofusion. Le procédé selon l'invention consiste à injecter dans un premier temps, préférentiellement à l'aide d'un système piezo électrique, le noyau dénudé de la cellule donneuse du côté opposé à la métaphase ovocytaire; puis dans un second temps à retirer par dépression négative la métaphase de l'ovocyte lors du retrait de la pipette d'injection, cette dernière étant extraite hors du cytoplasme avec un peu de la membrane plasmique au site d'entrée de la pipette. Ce procédé permet à la membrane plasmique de se sceller au site d'effraction de la pipette limitant ainsi considérablement la lyse ovocytaire. L'embryon est donc reconstruit dans un temps très court évitant de ce fait une activation ovocytaire prématurée, antérieure au transfert de noyau.

Pour l'étape c) du procédé selon l'invention, le milieu d'activation est avantageusement substantiellement exempt d'inhibiteur réversible du protéasome.

Les inducteurs d'activation des ovocytes reconstitués sont bien connus de l'homme du métier, notamment décrits par Hardcastle & al. (Designing Inhibitors of Cyclin-Dependent Linases, Annu. Rev. Pharmacol. Toxicol. 2002, 42:325-48). Selon un mode préférentiel de réalisation de l'invention, l'inducteur d'activation est la butyrolactone-1.

La quantité d'inducteur d'activation dans le milieu de culture de l'ovocyte reconstitué sera déterminée par l'homme du métier en fonction des propriétés spécifiques de chaque activateur. Pour la butyrolactone-1, la concentration de cet inhibiteur dans le milieu de culture sera de préférence comprise entre 25 et 150µM.

La cellule donneuse peut être n'importe quel type cellulaire de rat qui contient un génome ou du matériel génétique, tel que les cellules somatiques, les cellules germinales, les cellules embryonnaires telles les cellules souches pluripotentes, les cellules souches totipotentes, comme les cellules souches embryonnaires par exemple (cellules ES). Le terme « cellule somatique » se réfère à des cellules diploïdes différenciées. La cellule somatique de rat peut indifféremment provenir d'un animal, ou d'une culture de cellules ou de tissus ayant subi au moins un passage en culture et ayant été congelée ou non. Lorsque la cellule somatique dérive d'un animal, l'animal peut être à n'importe quel stade de développement, par exemple un embryon, un foetus ou un adulte.

Les cellules somatiques comprennent de préférence et de manière non limitative les fibroblastes (par exemple, les fibroblastes primaires), les cellules épithéliales, les cellules musculaires, les cellules cumulus, les cellules neurales, les cellules mammaires, les hépatocytes, les cellules de Langerhans, les cellules dérivées des trois feuillets embryonnaires qui permettent la formation d'un organisme en ecto, méso et endoderme. De préférence, les cellules somatiques donneuses sont des fibroblastes foetaux de rat. Les cellules somatiques peuvent être obtenues par exemple par dissociation de tissus par des moyens mécaniques ou enzymatiques (en général par l'utilisation de trypsine ou de protéases) pour obtenir une suspension cellulaire qui est en général cultivée jusqu'à l'obtention d'une monocouche cellulaire confluente. Les cellules somatiques peuvent être récoltées ou préparées pour la cryopréservation et maintenues congelées jusqu'à une utilisation ultérieure. Les cellules donneuses de noyaux sont indifféremment à l'état prolifératif ou à l'état de quiescence. L'état de quiescence qui correspond au stade Go/G1 du cycle cellulaire est obtenu chez les cellules en culture par inhibition de contact ou par privation en sérum (Whitfield *et al*., 1985) ou par des inhibiteurs du cycle cellulaire (Kues WA et al. 2002, Cell cycle synchronisation of porcine fetal fibroblasts : effect of sérum deprivation and reversible cell inhibitors). L'état prolifératif peut être considéré comme correspondant à tous les autres stades du cycle cellulaire.

De manière préférentielle, le noyau de la cellule donneuse est prélevé pour son insertion dans l'ovocyte récepteur lorsque sa chromatine est en mitose.

De manière avantageuse, l'embryon reconstitué est implanté dans l'oviducte ou l'utérus d'une femelle receveuse et, on laisse ledit embryon transféré s'implanter et se développer dans l'utérus de la dite femelle receveuse.

Les techniques de transfert d'embryons reconstitués dans des utérus de femelles receveuses sont bien connues de l'homme du métier.

Selon un mode particulier de réalisation de l'invention, l'embryon reconstitué de rat est transféré au stade 1 cellule.

Selon un autre mode particulier de réalisation de l'invention, l'embryon reconstitué de rat est transféré au stade 2 cellules.

Une fois transféré, l'embryon se développera avantageusement en foetus, puis en nouveau-né.

L'invention concerne aussi un procédé *in vitro* de clonage de rats par transfert nucléaire comprenant ou incluant un procédé selon l'invention décrit précédemment.

Selon un mode particulier de réalisation de l'invention, les cellules donneuses peuvent également avoir été manipulées génétiquement notamment par la dérégulation positive ou négative d'un gène ou par intégration d'un gène hétérologue. Les techniques de manipulation génétiques des cellules de mammifères sont bien connues de l'homme du métier.

Selon un mode particulier de réalisation de l'invention, l'embryon reconstitué est donc un embryon transgénique.

Le transgène, c'est-à-dire l'acide nucléique hétérologue intégré dans le génome de la cellule donneuse de rat n'est pas limité à une séquence particulière d'ADN. Il peut s'agir d'une séquence codant pour une protéine hétérologue, ou encore une séquence codant pour un ARN susceptible de bloquer la traduction d'un ARN produit par un gène endogène, comme par exemple un ARN antisens. La séquence d'acide nucléique du transgène, en particulier d'ADN, peut être d'une origine purement synthétique (par exemple réalisée en routine à partir d'un synthétiseur d'ADN), ou peut dériver de séquences d'ARNm par reverse transcription, ou peut dériver directement de séquences d'ADN génomique. Lorsque la séquence d'ADN dérive de séquences d'ARN par reverse transcription, celle-ci peut contenir ou non tout ou partie de séquences non codantes telles les introns, selon que la molécule d'ARN correspondante a ou non subi, partiellement ou totalement, un épissage. Le transgène peut être aussi petit que quelques centaines de paires de bases d'ADNc ou aussi large qu'une centaine de milliers de paires de bases d'un locus génique comprenant la séquence codante exonique-intronique et les séquences de régulation nécessaires à l'obtention d'une expression contrôlée de manière spatio-temporelle. Le segment d'ADN recombiné peut avoir une taille comprise entre 2,5 kb et 1 000 kb. Le segment d'ADN recombiné peut aussi être inférieurs à 2,5 kb ou supérieurs à 1 000 kb, permettant une transgénèse par Yac (Pook MA & al. 2001)

Le transgène ou la séquence d'ADN est de préférence sous forme native, c'est-à-dire dérivée directement d'une séquence d'ADN exogène naturellement présente dans une cellule animale. Cette séquence d'ADN sous forme native peut être modifiée par exemple par insertion de sites de restriction nécessaires au clonage et/ou par insertion de sites de recombinaison site-spécifiques (séquences lox et flp). Alternativement, la séquence d'ADN peut avoir été créée artificiellement *in vitro* par synthèse chimique mais aussi par les techniques de l'ADN recombinant, en associant par exemple des portions d'ADN génomique et d'ADNc.

Le transgène contient de préférence des séquences de régulation appropriées pour diriger et contrôler l'expression des gènes codant desdits polypeptides dans le ou les type(s) cellulaire(s) approprié(s). Par éléments contrôlant l'expression génique, on entend désigner toutes les séquences d'acide nucléique impliquées dans la régulation de l'expression génique c'est-à-dire essentiellement les séquences régulatrices de la transcription, de l'épissage, de la traduction. Parmi les séquences régulatrices de la transcription, il convient de citer la séquence promotrice minimale, les séquences amonts (par exemple, la boîte SP1, l'IRE pour «interferon responsive element»,...), les séquences activatrices (« enhancers »), éventuellement les séquences inhibitrices (« silencers »), les séquences insulateurs (« insulators »), les séquences d'épissage. Les éléments contrôlant l'expression génique permettent soit une expression constitutive, ubiquitaire, inductible, spécifique d'un type cellulaire (« tissu-spécifique ») ou spécifique d'une étape du développement. Ces éléments peuvent être ou non hétérologues à l'organisme, ou être naturellement présents ou non dans le génome de l'organisme. Il est évident qu'en fonction du résultat recherché, l'homme du métier choisira et adaptera les éléments de régulation de l'expression des gènes. Pour diriger l'expression du transgène dans un fluide biologique de l'animal, tel le lait, la séquence régulatrice de la transcription utilisée est sélectionnée dans les séquences promotrices des gènes actifs spécifiquement dans les cellules sécrétant ces fluides biologiques, telles les cellules des glandes mammaires par exemple pour diriger l'expression dans le lait. Parmi les fluides biologiques préférés, il convient de citer le lait, le sang, le sperme, l'urine.

L'utilisation d'un rat cloné transgénique pour la production de protéines recombinantes d'intérêt, est un objet de la présente description. La protéine recombinante d'intérêt peut être n'importe quelle protéine, par exemple une protéine thérapeutique telle que la α-, β-, δ-globine, les facteurs de coagulation sanguine (facteurs VIII et IX), les récepteurs de surface cellulaire, les.anticorps, les enzymes, etc... et autres protéines nécessaires pour par exemple corriger des défauts hérités ou acquis chez un patient.

La présente description concerne également l'utilisation d'un rat cloné, en particulier d'un rat cloné transgénique, comme modèle d'étude de pathologies humaines. A titre d'exemple de pathologies humaines, il convient de citer la mucoviscidose, l'athérosclérose, les cancers, les maladies du métabolisme, les pathologies oculaires. Les rats clonés peuvent également être employés dans toutes études neurobiologiques ou sur des modèles comportementaux.

De manière avantageuse, les ovocytes récepteurs et/ou les cellules donneuses proviennent de lignées de rat choisies parmi les lignées consanguines, les lignées hybrides et les lignées dites « outbred » non consanguines. De préférence, les ovocytes récepteurs et/ou cellules donneuses proviennent de lignées « outbred » non consanguines, comme par exemple les lignées Sprague Dawley ou Wistar.

Le procédé selon l'invention présente l'avantage de contrôler strictement et de façon réversible le processus d'activation ovocytaire en agissant en amont des processus biochimiques qui contrôlent l'activation et en prévenant la dégradation des composés macromoléculaires responsables de l'arrêt de l'ovocyte en métaphase et notamment des cyclines. Pour cela l'ovocyte est exposé in vitro à un(des) inhibiteur(s) du protéasome, un organite clé de la cellule : l'inhibition de son action protéolytique sur les cyclines permet de maintenir un taux élevé de l'activité kinasique MPF qui détermine l'arrêt en métaphase. La majorité des ovocytes peuvent ainsi être maintenus en métaphase de seconde division méiotique pendant plusieurs heures après leur récolte (tableau 1).

**Tableau 1 Inhibition de l'activation spontanée d'ovocytes de rat par exposition à un inhibiteur du protéasome (MG132)**

| ***% d'ovocytes activés après collecte (t=0)*** | | |
|---|---|---|
| | t=0 | t=210m |
| MG132 | 0% (0/68) | 33,8% (23/68) |
| Contrôle | 0% (0/46) | 76,1% (35/46) |

| | | |
|---|---|---|
| Répétitions, n= 2 | | |

Lorsque l'inhibiteur est retiré, les mécanismes de l'activation peuvent être déclenchés, l'ovocyte expulse le second globule polaire et peut initier un développement normal (tableau 2).

**Tableau 2 Réversibilité de l'inhibition de l'activation spontanée d'ovocytes de rat après leur exposition à un inhibiteur du protéasome (MG132) pendant 120min.**

| ***% d'embryons (développement parthenogénétiques* au *stade morula) obtenus après retrait du MG132 et induction du processus d'activation par le butyrolactone*** | |
|---|---|
| t=0 retrait du MG132 | t=120min |
| 0%(0/71) | 45,9% (34/74) |

| | |
|---|---|
| Répétitions, n= 5 | |

Les ovocytes conservent leurs propriétés mécaniques vis à vis de la micromanipulation ce qui permet la réalisation effective en routine des opérations requises pour le transfert de noyaux (tableau 3).

**Tableau 3 Clivage des embryons de rat reconstruits par transfert nucléaire après retrait du MG132 puis exposition_au BLI**

| ***% d'embryons* au *stade* 2 *cellules 24h après retrait du BLI*** | |
|---|---|
| Contrôle* | 66.5% (117/176) |
| MG132** | 89.2% (166/18) |

| | |
|---|---|
| *répétitions = 4 ; ** répétitions = 3 | |

Par le procédé selon l'invention, les ovocytes sont transférés dans un milieu ne contenant pas le(s) inhibiteur(s) juste avant de procéder à la reconstruction des embryons. Ceci permet d'éviter l'exposition directe des cellules donneuses de noyau à leur action. La reconstruction des embryons doit alors être effectuée très rapidement avant que le protéasome ne redevienne fonctionnel afin d'éviter une activation prématurée de l'ovocyte. Cette reconstruction rapide a pour but d'allonger la période pendant laquelle la chromatine condensée du noyau sera directement exposée aux facteurs ovocytaires qui permettent la reprogrammation de l'activité du noyau étranger. Il devient alors possible de reconstituer un plus grand nombre d'embryons pendant une période de temps donnée, et donc d'augmenter le nombre d'embryons transplantés par femelle receveuse pour un développement in vivo. Cette possibilité permet de pallier au faible taux d'implantation observé chez le rat après transfert de noyau (tableau 4).

**Tableau 4. Taux de développement foetal des embryons de rat reconstruits à partir d'ovocytes préalablement exposés à un inhibiteur du protéasome (MG132)**

| traitement | receveuses gestantes entre J12-22 | implantation (%) | Nb foetus (%) | |
|---|---|---|---|---|
| | | | total | viable |
| MG132 | 11 | 34 (5.4) | **19 (55.9)** | **16*(47.0)** |
| contrôle | 11 | 9(4.1) | **0** | **0** |

| | | | | |
|---|---|---|---|---|
| * 5 rats vivants à terme, 11 vivants à J12-J14 | | | | |

On constate dans ces conditions que le taux d'embryons implantés qui se développe en foetus devient significativement plus élevé. Ces foetus peuvent donner naissance à des ratons viables (tableau 4)

### Matériel et Méthode

### 1. Récolte des ovocytes de rattes

### Moyens :

- Matériel stérile de chirurgie
   o 1 pince recourbée à mords
   o 2 pinces fines droites
   o 1 paire de petits ciseaux et une paire de gros ciseaux
   o aiguille (23Gx1") + seringue (5ml)
- Loupes binoculaires + platines chauffantes
- pipettes de manipulation également utilisée pour enlever par aspiration refoulement dans la pipette la couronne de cellules qui à ce stade entoure l'ovocyte (« décoronisation »).
- boîtes Pétri 35 et 60mm de diamètre

### Solutions :

- Milieu M2 (Hogan & al.1994 In Manipulating the Mouse Embryo)
- Milieu RECMB2*-MG132 (MG132 = 1.25µM)
- Milieu M2-Hyaluronidase-MG132 (MG132=1,25µM ; Hyaluronidase=2,5mg/ml)
- Milieu M2-MG132 (MG132=5,00µM)
   * RECMB2= milieu mR1ECM-BSA (Oh & al. (1998) Biol. Reprod.), avec BSA = 4mg/ml et NaCl=100-130mM.

### Mode opératoire :

La veille de récolte:
   - allumer les platines chauffantes et mettre du milieu M2 et de l'huile de paraffine à 37°C.
   - préparer les boîtes de milieux de culture (RECMB) et les mettre à l'incubateur 37°C, 5% CO2
      1. Les boîtes utilisées pour la récolte des ovaires-oviductes (M2-MG132 et M2-hyaluronidase-MG132) sont préparées avec du milieu et M2 et de l'huile de paraffine à 37°C et sont installées sur les platines chauffantes à 37°C au moins ½ heure avant la récolte.
         Préparer :
         - 2 boîtes de Pétri 35mm contenant 1.5 à 2ml de milieu M2-MG132 sans huile par femelle.
         - 1 boîte de M2 supplémentaire sans huile pour le rinçage-décoronisation post hyaluronidase
         - 1 boîte de M2/hyaluronidase avec huile pour 2 femelles
         - Toutes les boîtes sont laissées sur les platines chauffantes pour toute la durée de la récolte.
      2. Les rattes sont sacrifiées une par une par dislocation cervicale.
      3. Passer les poils à l'alcool 70%.
      4. Pratiquer une ouverture ventrale (peau + muscle) en remontant sur les flancs à l'aide de la grosse paire de ciseaux et de la pince recourbée.
      5. L'ensemble ovaire et oviducte est prélevé bilatéralement le plus rapidement possible après le sacrifice (environ 1-2 min) avec une seconde paire de pinces et de ciseaux. En suivant la trompe utérine remonter jusqu'à l'ovaire et récupérer l'ensemble ovaire-oviducte. Transférer le dans une boîte de M2.
      6. Lorsque les 2 ensembles ovaire-oviducte ont été récupérés, percer l'ampoule de chaque oviducte avec des aiguilles stériles montées sur des seringues de 5ml pour libérer les cumulus.
      7. Récolter, les cumulus au pipetman (P1000) et les transférer soit dans une seconde boîte de M2 ou ils pourront être stockés en attendant que les cumulus des autres femelles soient récoltés soit dans la goutte de M2-hyaluronidase-MG132 pour si la décoronisation est effectuée immédiatement.
      8. Transférer au pipetman (P1000) les cumulus dans une goutte de M2-hyaluronidase-MG132 (500µl). Attendre quelques secondes que l'enzyme commence à agir puis aspirer-refouler les cumulus à la pipette pour accélérer leur dissociation.
      9. Une fois les cumulus dissociés, transférer les ovocytes dans une boîte de M2 (rinçage) et finir de les « décoroniser » mécaniquement si les cellules du cumulus sont trop nombreuses avec la pipette de décoronisation.
      10. Les ovocytes « décoronisés » sont transférés dans une goutte de milieu de culture RECMB2-MG132.

Il faut éviter au maximum les chocs thermiques pour les ovocytes donc manipuler assez rapidement et toujours à 37°C.

### 2. Production d'embryons par transfert du noyau d'une cellule donneuse dans le cytoplasme énucléé d'un ovocyte

### Moyens :

- Microscope inversé avec contraste d'interférence différentiel et objectifs x4 et x20
- Micromanipulateurs motorisés Eppendorf droit et gauche
- Chambre de micromanipulation des ovocytes et cellules donneuses de noyau
- Pipettes pour la manipulation des ovocytes
- Micropipettes de maintien de l'ovocyte
- Micropipettes d'énucléation-injection
- Système Piezo-Drill

### Solutions :

- Milieu M2
- Milieu RECMB2
- Milieu RECMB2-MG132 (MG132=1,25µM)
- Milieu RECMB2-BLI (BLI=150µM)
- Huile paraffine légère SIGMA pour couvrir le milieu des boîtes de culture et de la chambre de micromanipulation
- Huile paraffine lourde SIGMA pour le système de micro-injection
- Fluorinert

### Mode opératoire :

Tous les milieux de culture (RECM) avec ou sans drogues sont préparées la veille, recouvert d'huile de paraffine légère et gardés à l'incubateur 37°C, 5%CO2.
1. Récolter les ovocytes selon le protocole décrit précédemment.
2. Assembler la chambre de micromanipulation avec une lamelle adaptée.
3. Déposer au pipetman environ 500µl de M2 sur la lamelle en formant une goutte allongée et recouvrir d'huile de paraffine légère.
4. Remplir la micropipette d'énucléation-injection de Fluorinert à partir de son extrémité non étirée à l'aide d'une seringue de 1ml dont l'aiguille est connectée à un tuyau de caoutchouc. Veiller à ne pas injecter d'air dans la micropipette.
   Vérifier qu'il y ait assez d'huile dans l'injecteur (seringue eppendorf). Faire couler de l'huile du porte pipette avant d'installer la pipette afin de vérifier qu'aucune bulle d'air ne se trouve piégée dans le système.
   Installer les pipettes de contention et d'énucléation-injection sur leurs porte-pipettes respectifs.
5. Positionner les pipettes de façon à ce que leurs extrémités soient horizontales, c'est-à-dire parallèle à la lamelle de la chambre de micromanipulation, et en regard l'une de l'autre.
6. Transférer les ovocytes et les cellules dans la chambre de micro injection à l'aide d'une pipette de manipulation des ovocytes.
7. Maintenir un ovocyte sur la pipette de maintien afin que la déformation de la membrane plasmique sous laquelle se situe la métaphase ovocytaire se situe entre 2 et 3h ou entre 3 et 4h.
8. Appliquer la pipette d'énucléation-injection sur la zone pellucide à 3h. Vérifier que la pipette soit exactement dans le même plan que la zone pellucide. Aspirer légèrement la zone pellucide et actionner le système piezo (réglages 1) pour percer celle ci. Lorsque le système est parfaitement réglé la ZP est percée en qq secondes selon un parfait emporte-pièce.
9. Aspirer ensuite une cellule dont la morphologie correspond aux critères recherchés dans la pipette d'énucléation-injection et casser sa membrane plasmique par plusieurs allers-retours dans la pipette ou à l'aide du piezo-Drill (quelques impulsions suffisent à réglages 2). Maintenir la cellule proche de l'extrémité de la micropipette.
10. Pénétrer dans l'espace périvitellin de l'ovocyte par l'orifice préalablement percé, appliquer la pipette contre la membrane plasmique et repousser la cellule donneuse à l'extrémité de la pipette puis enfoncer la pipette jusqu'à l'extrémité opposé de l'ovocyte sans toutefois atteindre la membrane plasmique opposée.
11. Casser la membrane plasmique à l'aide d'une ou de plusieurs impulsions (1 à 3) du Piezo-Drill (réglages 2).
12. Injecter la cellule dans le cytoplasme de l'ovocyte avec le minimun de milieu possible.
13. Retirer doucement la pipette d'énucléation-injection et refermer l'ovocyte en aspirant les 2 extrémités de la membrane plasmique rompue et la métaphase ovocytaire (énucléation).
14. Transférer les embryons reconstruits dans une micro-goutte (50µl) de RECMB2.
15. L'activation ovocytaire est déclenchée ou prolongée par transfert des ovocytes dans une micro-goutte (20µl) de RECMB2-BLI pour une durée d'activation généralement comprise entre 1 et 2h.
16. Les ovocytes sont ensuite rincés dans 4-5 gouttes (50µl) de RECMB2 pour être cultivés dans le même milieu en attendant d'être transférés dans une femelle pseudogestante ou de prolonger la culture in vitro.

### Références

- Baguisi et al. (1999) Nature Biotechnol. 17, 456-461.
- Campbell & al., Nuclear transfer in practice, School of Biosciences, University of Nottingham, Leicestershire, United Kingdom.
- Chesne et al. (2002) Nature Biotechnol. 20, 366-369.
- Hardcastle & al. (2002) Annu. Rev. Pharmacol. Toxicol. 42, 325-48.
- Hayes & al. (2001) Physiol. Genomics 5, 193-203.
- Hee Lee & Goldberg (1998) Trends in Cell Biology 8, 397-403
- Hirabayashi & al. (2003) Cloning and Stem Cells 5(1), 35-41.
- Hirabayashi & al. (2003) J. of Reproduction and Development 49(2), 121-6.
- Hogan B & al. Recovery, culture and transfer of embryos and germ cells. In: Manipulating the Mouse Embryo: A Laboratory Manual, 2nd ed. Plainview, NY: Cold Spring Harbor Laboratory Press 1994; 173-181
- lannaccone & al. (2001) Zygote 9, 135-43.
- Josefnerg & al. (2000) Biol. Reprod. 62, 1270-77.
- Kues WA et al. (2002) Biol Reprod., 62, 412-419.
- Miyoshi et al. (1997) Biol. Reprod. 56, 180-185.
- Oh SH et al. (1998) Biol. Reprod., 59, 884-889.
- Polejaeva et al. (2000) Nature 407, 86-90.
- Pook MA et al. (2001) Neurogenetics, 3, 185-193.
- Wakayama et al. (1998) Nature 394, 369-374.
- Wells et al. (1999) Biol. Reprod. 60, 996-1005.
- Whitfield et al. (1985) Control of Animal Cell Proliferation 1, 331-365.
- Wilmut et al. (1997) Nature 385, 810-813.
- Zernicka-Goetz M. (1991) Molecular Reproduction and Dévelopment 28, 169-76.

## Revendications

1. Procédé *in vitro* de production d'embryons de rats reconstitués par transfert nucléaire comprenant les étapes suivantes :
a) prélèvement d'un ovocyte de ratte et maintien dans un milieu approprié ;
b) insertion d'un noyau de cellule de rat dans l'ovocyte receveur de ratte précédemment maintenu in vitro pour obtenir un ovocyte reconstitué ;
c) activation de l'ovocyte reconstitué obtenu à l'étape b pour obtenir un embryon reconstitué ;
**caractérisé en ce que**
- l'ovocyte receveur de ratte prélevé est maintenu à un stade métaphasique de la seconde division méiotique par maintien dans un milieu approprié comprenant un inhibiteur réversible du protéasome, et **en ce que**
- l'ovocyte reconstitué est activé par exposition dans un milieu approprié contenant un inducteur de l'activation.

2. Procédé selon la revendication 1, **caractérisé en ce que** l'inhibiteur réversible du protéasome est choisi parmi le Cbz-LLL-H (MG132), le peptide boronate Cbz-LLL-B(OH)2, le vinyl sulfone Cbz-LLL-Vs, la lactacystine et la bêta-lactone, la leupeptine, le MG101, le MG115, le MG262 et le PSI (Cbz-ile-glu).

3. Procédé selon la revendication 2, **caractérisé en ce que** l'inhibiteur réversible du protéasome est le MG132.

4. Procédé selon l'une des revendications 1 à 3, **caractérisé en ce que** la concentration en inhibiteur réversible du protéasome dans le milieu de maintien des ovocytes receveurs de ratte est comprise entre 1 et 10µM.

5. Procédé selon l'une des revendications 1 à 4, **caractérisé en ce que** le temps d'exposition de l'ovocyte receveur de ratte à l'inhibiteur réversible du protéasome dans le milieu de maintien est inférieur à 10 heures, de préférence compris entre 1 et 6 heures.

6. Procédé selon l'une des revendications 1 à 5, **caractérisé en ce que** pour l'étape b) de transfert nucléaire, le milieu de culture de l'ovocyte receveur de ratte est substantiellement exempt d'inhibiteur réversible du protéasome.

7. Procédé selon l'une des revendications 1 à 6, **caractérisé en ce que** le transfert nucléaire comprend une étape d'enlèvement du noyau de l'ovocyte receveur de ratte, suivi d'une étape d'introduction du noyau de cellule donneuse de rat dans l'ovocyte énucléé.

8. Procédé selon l'une des revendications 1 à 6, **caractérisé en ce que** le transfert nucléaire comprend une étape d'introduction du noyau de cellule donneuse de rat dans l'ovocyte receveur de ratte suivi d'une étape d'enlèvement du noyau de l'ovocyte receveur.

9. Procédé selon l'une des revendications 6 ou 7, **caractérisé en ce que** le transfert du noyau de cellule donneuse de rat dans le cytoplasme de l'ovocyte receveur de ratte est effectué par micro-injection et/ou l'enlèvement du noyau de l'ovocyte receveur est effectué par micropipetage.

10. Procédé selon l'une des revendications 1 à 9, **caractérisé en ce que** le milieu approprié contenant un inducteur de l'activation est substantiellement exempt d'inhibiteur réversible du protéasome.

11. Procédé selon l'une des revendications 1 à 10, **caractérisé en ce que** l'inducteur d'activation est la butyrolactone-1.

12. Procédé selon les revendications 1 à 11, **caractérisée en ce que** ledit embryon reconstitué de rat est un embryon transgénique.

13. Procédé selon l'une des revendications 1 à 12, **caractérisé en ce que** l'on transfère ledit embryon reconstitué de rat dans l'oviducte ou l'utérus d'une femelles de rat receveuse et, on laisse ledit embryon transféré s'implanter et se développer dans l'utérus de la dite femelle receveuse.

14. Procédé selon la revendication 13, **caractérisée en ce que** ledit embryon reconstitué de rat est transféré au stade 1 cellule.

15. Procédé selon la revendication 13, **caractérisée en ce que** ledit embryon reconstitué de rat est transféré au stade 2 cellules.

16. Procédé selon les revendications 13 à 15 **caractérisée en ce que** ledit embryon transféré de rat se développe en foetus.

17. Procédé selon la revendication 16 **caractérisée en ce que** ledit foetus de rat se développe en nouveau-né.

18. Procédé *in vitro* de clonage de rats par transfert nucléaire comprenant ou incluant un procédé selon l'une quelconque des revendications 1 à 17.

## Claims

1. *In vitro* method for producing reconstituted rat embryos by nuclear transfer, comprising the following steps:
a) removal of a rat oocyte and maintenance in an appropriate medium;
b) insertion of a rat cell nucleus into the recipient rat oocyte previously maintained *in vitro* in order to obtain a reconstituted oocyte;
c) activation of the reconstituted oocyte obtained in step b in order to obtain a reconstituted embryo; **characterised in that**
- the recipient rat oocyte removed is maintained at the metaphase stage of the second meiotic division by maintenance in an appropriate medium including a reversible proteasome inhibitor, and **in that**
- the reconstituted oocyte is activated by exposure in an appropriate medium containing an activation inducer.

2. Method as claimed in claim 1, **characterised in that** the reversible proteasome inhibitor is chosen from among Cbz-LLL-H (MG132), peptide boronate Cbz-LLL-B-(OH)2, vinyl sulfone Cbz-LLL-Vs, lactacystin and beta-lactone, leupeptin, MG101, MG115, MG262 and PSI (Cbz-ile-glu).

3. Method as claimed in claim 2, **characterised in that** the reversible proteasome inhibitor is MG132.

4. Method as claimed in one of claims 1 to 3, **characterised in that** the reversible proteasome inhibitor concentration in the medium for maintenance of the recipient rat oocytes is between 1 and 10 µM.

5. Method as claimed in one of claims 1 to 4, **characterised in that** the exposure time of the recipient rat oocyte to the reversible proteasome inhibitor in the maintenance medium is less than 10 hours, preferably between 1 and 6 hours.

6. Method as claimed in one of claims 1 to 5, **characterised in that**, for the nuclear transfer step b), the culture medium of the recipient rat oocyte is substantially free of reversible proteasome inhibitor.

7. Method as claimed in one of claims 1 to 6, **characterised in that** the nuclear transfer includes a step for removal of the recipient rat oocyte nucleus, followed by a step for introduction of the donor rat cell nucleus into the enucleated oocyte.

8. Method as claimed in one of claims 1 to 6, **characterised in that** the nuclear transfer includes a step for introduction of the donor rat cell nucleus into the recipient rat oocyte, followed by a step for removal of the recipient oocyte nucleus.

9. Method as claimed in one of claims 6 or 7, **characterised in that** the transfer of the donor rat cell nucleus into the cytoplasm of the recipient rat oocyte is carried out by micro-injection and/or the removal of the recipient oocyte nucleus is carried out by micropipetting.

10. Method as claimed in one of claims 1 to 9, **characterised in that** the appropriate medium containing an activation inducer is substantially free from reversible proteasome inhibitor.

11. Method as claimed in one of claims 1 to 10, **characterised in that** the activation inducer is butyrolactone-1.

12. Method as claimed in claims 1 to 11, **characterised in that** said reconstituted rat embryo is a transgenic embryo.

13. Method as claimed in one of claims 1 to 12, **characterised in that** said reconstituted rat embryo is transferred into the oviduct or uterus of a recipient female rat and said transferred embryo is allowed to become implanted and to develop in the uterus of said recipient female.

14. Method as claimed in claim 13, **characterised in that** said reconstituted rat embryo is transferred at the 1-cell stage.

15. Method as claimed in claim 13, **characterised in that** said reconstituted rat embryo is transferred at the 2-cell stage.

16. Method as claimed in claims 13 to 15, **characterised in that** said transferred rat embryo develops into a foetus.

17. Method as claimed in claim 16, **characterised in that** said rat foetus develops into a neonate.

18. *In* vitro method for cloning rats by nuclear transfer, comprising or including a method as claimed in any of claims 1 to 17.

## Patentansprüche

1. *In* vitro-Verfahren zur Erzeugung von wiederhergestellten Rattenembryonen durch Zellkerntransfer, umfassend die folgenden Schritte:
a) Entnehmen eines Ratten-Oozyten und Erhalten in einem geeigneten Medium;
b) Einschleusen eines Rattenzellkerns in den Ratten-Empfängeroozyten, der zuvor in vitro erhalten wurde, um einen wiederhergestellten Oozyten zu erzielen;
c) Aktivieren des wiederhergestellten Oozyten, der in Schritt b erzielt wurde, um einen wiederhergestellten Embryo zu erzielen;
**dadurch gekennzeichnet, dass**
- der entnommene Ratten-Empfägeroozyt in einem metaphasichen Stadium der zweiten Meioseteilung in einem angemessenen Medium erhalten wird, umfassend einen reversiblen Proteasomhemmer und **dadurch**, dass
- der wiederhergestellte Oozyt durch Aussetzung in einem geeigneten Medium, umfassend einen Aktivierungsinduktor, aktiviert wird.

2. Verfahren nach Anspruch 1, **dadurch gekennzeichnet, dass** der reversible Proteasomhemmer unter Cbz-LLL-H (MG132), Peptidboronat Cbz-LLL-B(OH)2, Vinylsultfon Cbz-LLL-Vs, Lactacystin und Beta-Lakton, Leupeptin, MG101, MG115, MG262 und PSI (Cbz-ile-gtu) ausgewählt wird.

3. Verfahren nach Anspruch 2, **dadurch gekennzeichnet, dass** der reversible Proteasomhemmer MG132 ist.

4. Verfahren nach einem der Ansprüche 1 bis 3, **dadurch gekennzeichnet, dass** die Konzentration des reversiblen Proteasomhemmers im Erhaltungsmedium des Ratten-Empfängeroozyten zwischen 1 und 10 µM liegt.

5. Verfahren nach einem der Ansprüche 1 bis 4, **dadurch gekennzeichnet, dass** die Zeit der Aussetzung des Ratten-Empfängeroozyten an den reversiblen Proteasomhemmer im Erhaltungsmedium weniger als 10 Stunden, vorzugsweise zwischen 1 und 6 Stunden beträgt.

6. Verfahren nach einem der Ansprüche 1 bis 5, **dadurch gekennzeichnet, dass** für den Schritt b) des Zellkerntransfers das Kulturmedium des Ratten-Empfängeroozyten im Wesentlichen frei vom reversiblen Proteasomhemmer ist.

7. Verfahren nach einem der Ansprüche 1 bis 6, **dadurch gekennzeichnet, dass** der Zellkerntransfer einen Schritt des Entnehmens des Kerns des Ratten-Empfängeroozyten umfasst, gefolgt von einem Schritt des Einschleusens des Kerns der Ratten-Spenderzelle in den entnukleirten Oozyten.

8. Verfahren nach einem der Ansprüche 1 bis 6, **dadurch gekennzeichnet, dass** der Zellkerntransfer einen Schritt des Einschleusens des Kerns der Ratten-Spenderzelle in den Ratten-Empfängeroozyten umfasst, gefolgt von einem Schritt des Entnehmens des Kerns des Empfangeroozyten.

9. Verfahren nach einem der Ansprüche 6 oder 7, **dadurch gekennzeichnet, dass** der Transfer des Kerns der Ratten-Spenderzelle in das Zytoplasma des Ratten-Empfängeroozyten durch Mikroinjektion und/oder die Entnahme des Kerns des Empfängeroozyten durch Mikropipettierung durchgeführt wird.

10. Verfahren nach einem der Ansprüche 1 bis 9, **dadurch gekennzeichnet, dass** das geeignete Medium, das einen Reaktivierungsinduktor enthält, im Wesentlichen frei vom reversiblen Proteasomhemmer ist.

11. Verfahren nach einem der Ansprüche 1 bis 10, **dadurch gekennzeichnet, dass** der Aktivierungsinduktor das Butyrolacton-1 ist.

12. Verfahren nach Anspruch 1 bis 11, **dadurch gekennzeichnet, dass** der wiederhergestellte Ratten-Embryo ein transgener Embryo ist.

13. Verfahren nach einem der Ansprüche 1 bis 12, **dadurch gekennzeichnet, dass** der wiederhergestellte Ratten-Embryo in den Eileiter oder den Uterus einer weiblichen Empfängerratte übertragen wird und der übertragene Embryo im Uterus der weiblichen Empfängerratte der Implantierung und Entwicklung überlassen wird.

14. Verfahren nach Anspruch 13, **dadurch gekennzeichnet, dass** der wiederhergestellte Ratten-Embryo im Stadium mit 1 Zelle übertragen wird.

15. Verfahren nach Anspruch 13, **dadurch gekennzeichnet, dass** der wiederhergestellte Ratten-Embryo im Stadium mit 2 Zellen übertragen wird.

16. Verfahren nach Anspruch 13 bis 15, **dadurch gekennzeichnet, dass** sich der übertragene Ratten-Embryo zu einem Fötus entwickelt.

17. Verfahren nach Anspruch 16, **dadurch gekennzeichnet, dass** sich der Ratten-Fötus zu einem Neugeborenen entwickelt.

18. In vitro-Verfahren zur Klonierung von Ratten durch Zellkerntransfer, umfassend oder enthaltend ein Verfahren nach einem der Ansprüche 1 bis 17.
